(19) Europäisches Patentamt
European Patent Office
Office européen des brevets

(11) Veröffentlichungsnummer : **0 402 722 B1**

(12) # EUROPÄISCHE PATENTSCHRIFT

(45) Veröffentlichungstag der Patentschrift : **26.01.94 Patentblatt 94/04**

(51) Int. Cl.$^5$ : **C07D 231/12**

(21) Anmeldenummer : **90110517.1**

(22) Anmeldetag : **02.06.90**

(54) **Verfahren zur Herstellung von Pyrazol und dessen Derivaten.**

(30) Priorität : **10.06.89 DE 3918979**

(43) Veröffentlichungstag der Anmeldung : **19.12.90 Patentblatt 90/51**

(45) Bekanntmachung des Hinweises auf die Patenterteilung : **26.01.94 Patentblatt 94/04**

(84) Benannte Vertragsstaaten : **AT BE CH DE DK ES FR GB IT LI NL**

(56) Entgegenhaltungen :
**EP-A- 0 237 762**
**EP-A- 0 290 991**

(73) Patentinhaber : **BASF Aktiengesellschaft**
**Carl-Bosch-Strasse 38**
**D-67063 Ludwigshafen (DE)**

(72) Erfinder : **Merkle, Hans Rupert, Dr.**
**Brahmsstrasse 4**
**D-6700 Ludwigshafen (DE)**
Erfinder : **Fretschner, Erich**
**Hirtweg 76**
**D-6918 Neckarsteinach (DE)**

EP 0 402 722 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Pyrazol

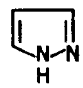

und dessen Derivaten aus 2-Pyrazolin

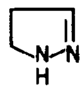

bzw. dessen Derivaten.

Es ist bekannt, 2-Pyrazolin mit Chlor, Alkalimetall- oder Erdalkalimetallhypochloriten (DE-A 30 35 395), mit Schwefel oder Selen (DE-A 30 29 160) oder mit wäßrigem Wasserstoffperoxid (DE-A 34 15 385) zum Pyrazol zu dehydrieren. Weiterhin sind die thermische Gasphasendehydrierung von 2-Pyrazolin an Palladium- oder Platinkontakten (DE-A 32 09 148) sowie die Thermolyse von N-Sulfonyl-2-pyrazolin zum Pyrazol (DE-A 30 35 394) bekannt.

Diese Verfahren sind jedoch technisch unbefriedigend, sei es, daß sie die Verwendung von sehr aggressiven Oxidationsmitteln oder teuren Katalysatoren erfordern, daß sie mit der Bildung giftiger Nebenprodukte wie Schwefel- und Selenwasserstoff verbunden sind oder daß sie sich nicht zur Herstellung von N-substituierten Pyrazolen eignen.

Der Erfindung lag daher die Aufgabe zugrunde, Pyrazol und dessen Derivate auf technisch einfachere und wirtschaftlichere Weise zugänglich zu machen.

Entsprechend dieser Aufgabe wurde ein Verfahren zur Herstellung von Pyrazol

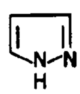

und dessen Derivaten durch Dehydrierung von 2-Pyrazolin

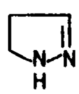

bzw. dessen Derivaten gefunden, das dadurch gekennzeichnet ist, daß man die Umsetzung mittels Schwefelsäure in Gegenwart von Jod oder einer Jod oder Jodwasserstoff freisetzende Verbindung bei einer Temperatur von 50°C bis 250°C vornimmt.

Das Verfahren beruht vermutlich auf dem Prinzip, daß das Jod als dehydrierendes Agens fungiert und im Laufe der Umsetzung in Jodwasserstoff übergeht. Der Jodwasserstoff kann sodann von der Schwefelsäure wieder zu Jod oxidiert werden, wodurch sich erklärt, daß man mit katalytischen Mengen des Jodes bzw. der Jodverbindung auskommmt.

Jod bzw. die Jodverbindung wird bei dieser Umsetzung im allgemeinen in Mengen von 0,01 bis 10 mol-% pro Mol des 2-Pyrazolins, vorzugsweise 0,05 bis 5 mol-% und insbesondere 0,1 bis 2 mol-% eingesetzt.

Sofern man von vornherein eine Jodverbindung einsetzt, so ist anzunehmen, daß sich zunächst Jod bildet und sich dann der geschilderte Zyclus anschließt.

Aus dem vorstehend geschilderten Katalysezyclus folgt, daß die Schwefelsäure als Oxidationsmittel für Jodwasserstoff fungiert und in einer entsprechenden Konzentration von nicht weniger als 30 Gew.-%, vorzugsweise von 45 bis 95 Gew.-% vorliegen soll. Eine entsprechende Konzentration der Schwefelsäure kann unter den Reaktionsbedingungen auch dadurch erreicht werden, daß man überschüssiges Wasser fortlaufend aus dem Reaktionsgemisch entfernt.

Neben elementarem Jod eignen sich als Katalysatoren auch Jodverbindungen wie Jodwasserstoff; Alkalimetall- und Erdalkalimetalljodide wie Lithiumjodid, Natriumjodid, Kaliumjodid, Cäsiumjodid, Magnesiumjodid und Calziumjodid sowie sonstige Metalljodide; es können auch andere anorganische Jodverbindungen wie Erdalkalimetall- und Alkalimetallhypojodide, -jodite, -jodate -und -perjodate oder organische Jodverbindungen wie Alkyljodide, z.B. Methyljodid zur Anwendung kommen.

Man führt die Dehydrierung im allgemeinen bei Normaldruck bei Temperaturen von 50°C bis 250°C, vorzugsweise 70°C bis 200°C, insbesondere 90°C bis 180°C, durch.

Es ist jedoch auch möglich, die Umsetzung bei erhöhtem Druck in geringer konzentrierter Schwefelsäure oder bei entsprechend erhöhter Temperatur bzw. bei verringertem Druck in höher konzentrierter Schwefelsäure oder bei entsprechend niedrigerer Temperatur durchzuführen.

Die Reaktion wird im allgemeinen so durchgeführt, daß alle Reaktionspartner im Reaktionsgefäß vereinigt und sodann gemeinsam auf Reaktionstemperatur erwärmt werden. Man kann aber auch die Reaktanden getrennt oder als Mischung in ein vorgeheiztes Reaktionsgefäß eintragen oder einen Teil der Reaktanden bei Reaktionstemperatur vorlegen und die oder den weiteren Reaktanden zugeben.

Das der Reaktion entzogene abdestillierte Wasser enthält den größten Teil des eingesetzten Jodids als Jodwasserstoff, welcher in die Reaktionsmischung zurückgeführt werden kann.

Nach dem Abkühlen der Reaktionsmischung kristallisiert das Pyrazolderivat als Schwefelsäuresalz.

Zur Aufarbeitung neutralisiert man die erhaltene Reaktionsmischung, z.B. mit Natronlauge, Ammoniak oder anderen anorganischen oder organischen Basen und extrahiert das neutrale Gemisch mehrfach mit einem inerten organischen, nicht mit Wasser mischbaren Lösungsmittel. Nach Trocknung des organischen Extraktes und Eindampfen zur Trockne erhält man die entsprechenden Pyrazole in 85 bis 95 %iger Reinheit, die zur Verbesserung der Reinheit destilliert oder umkristallisiert werden können.

Das erfindungsgemäße Verfahren eignet sich zur Herstellung von Pyrazol und substituierten Pyrazolen aus den entsprechenden 2-Pyrazolinderivaten, insbesondere solchen der allgemeinen Formel Ia,

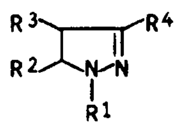

Ia

in welcher $R^1$ bis $R^4$ Wasserstoff oder C- organische Reste bedeuten, wobei die Natur der Substituenten nach den bisherigen Erkenntnissen nur von geringem Einfluß ist.

Als Reste $R^1$ bis $R^4$ kommen neben Wasserstoff vorzugsweise ein bis drei der folgenden Gruppen in Betracht:

$C_1$-$C_8$-Alkyl, insbesondere $C_1$-$C_4$-Alkyl wie Methyl, Ethyl, Propyl, 1-Methylethyl, Butyl, 1-Methylpropyl, 2-Methylpropyl und 1,1-Dimethylethyl; $C_3$-$C_8$-Cycloalkyl wie insbesondere Cyclopentyl, Cyclohexyl und Cycloheptyl; Aralkyl wie insbesondere durch Phenyl substituiertes $C_1$-$C_4$-Alkyl wie vorstehend genannt; Aryl wie insbesondere Phenyl.

Diese Reste können ihrerseits durch Heteroatome wie Stickstoff, Sauerstoff und Schwefel unterbrochen sein oder weitere inerte Substituenten wie Halogen, Nitro, Sulfonyl, $C_1$-$C_4$-Alkylsulfonyl, Arylsulfonyl und Carboxyl tragen, soweit sie sich unter den Reaktionsbedingungen inert verhalten.

Die als Ausgangsverbindungen dienenden 2-Pyrazoline sind bekannt oder nach bekannten Methoden erhältlich, wobei sich die Kondensation einer Verbindung mit Acrolein- bzw. Vinylalkylketon-Struktur und einem Hydrazin besonders empfiehlt (Behr et al, The Chemistry of Heterocyclic Compounds, Vol.22, Chapter 7; DE-A 3423930).

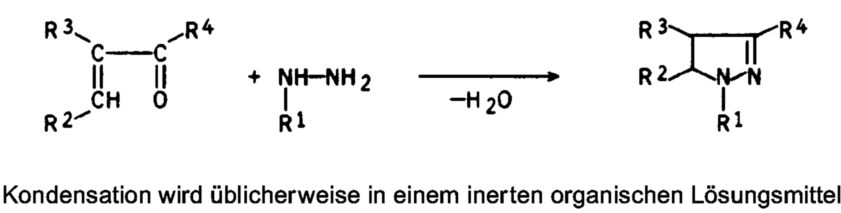


Ia

Diese Kondensation wird üblicherweise in einem inerten organischen Lösungsmittel oder in wäßriger Lösung in Gegenwart von Basen oder Säuren durchgeführt.

Im Hinblick auf das neue Verfahren ist es besonders bevorzugt, bereits diese Kondensationsreaktion mit Schwefelsäure vorzunehmen, wobei der Dehydrierungskatalysator Jod bzw. die Jodverbindung von vornherein zugesetzt wird und die Zwischenstufe des 2-Pyrazolins nicht isoliert wird.

Die Umsetzung erfolgt in diesem Fall zweckmäßigerweise derart, daß man 0,65 bis 1,25 mol-Äquivalent

des entsprechenden Acrolein/Vinylalkylketon-Derivats mit 1 mol-Äquivalent des Hydrazins unter den vorstehend geschilderten Bedingungen in Schwefelsäure in Gegenwart von Jod bzw. einer Jodverbindung umsetzt.

Anstelle der Vinylalkylketone können auch deren Vorläufer, z.B. (β-Hydroxy)ethylalkylketone eingesetzt werden.

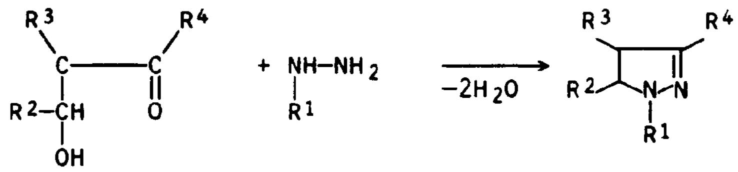


Es ist auch möglich, die (β-Hydroxy)-ethylketone selbst in situ z.B. aus Aceton und Formaldehyd herzustellen.

Eine weitere Möglichkeit zur Herstellung der 2-Pyrazoline besteht in der Umsetzung einer Verbindung mit Glycerin-Struktur und einem entsprechenden Hydrazin.

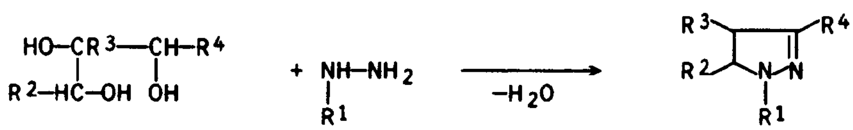


Wie bereits für die Kondensation der Acrolein/Vinylalkylketon-Derivats mit Hydrazin-Derivaten beschrieben, kann man auch die Kondensation der Glycerin-analogen Verbindungen mit Hydrazin-Derivaten in Schwefelsäure in Gegenwart von Jod bzw. der Jodverbindung vornehmen, wobei bei geeigneter Reaktionstemperatur die Zwischenstufe des 2-Pyrazolins direkt dehydriert wird.

Verfahrensbeispiele

1. Umsetzung von 2-Pyrazolin zu Pyrazol

270 g 80 Gew.-%ige Schwefelsäure wurden innerhalb von 90 min bei 155°C mit einer Mischung aus 145,8 g 48 Gew.-%iger wäßriger Lösung von Pyrazolin (1 mol) und 2 g Natriumjodid (0,0134 mol) versetzt. Durch Abdestillation von Wasser wurde die Temperatur der Reaktionsmischung bei 155°C gehalten. Nach vollständiger Zugabe wurde weitere 30 min bei 155°C belassen und anschließend auf 60°C abgekühlt.

Die so erhaltene Reaktionsmischung wurde mit 20 %iger Natronlauge neutralisiert. Man erhielt das Pyrazol durch Extraktion der Reaktionsmischung mit einem inerten nicht mit Wasser mischbaren organischen Lösungsmittel in üblicher Weise.
Ausbeute 69,5 g Festkörper von 93,1 %-iger Reinheit (GC-Analyse) entsprechend 95,2 %, bezogen auf das eingesetzte 2-Pyrazolin.

2. Allgemeine Arbeitsvorschrift zur Herstellung von Pyrazolen aus Hydrazinen und Acroleinen, Vinylalkylketonen bzw. (β-Hydroxy)ethylalkylketonen oder Glycerinen

2.1
n g einer c Gew.-%igen Schwefelsäure wurden unter Kühlung gleichzeitig oder nacheinander mit Hydrazinhydrat oder einem Hydrazin $H_2N\text{-}NHR^1$ (A-3), einem Acrolein/Vinylalkylketon $R^2CH{=}CR^3\text{-}CO\text{-}R^4$ (A-1), bzw. einem (β-Hydroxy)ethylalkylketon $R^2\text{-}CH(OH)\text{-}CR^3(H)\text{-}CO\text{-}R^4$(A-1∗) bzw. einem Glycerin $R^2\text{-}CH(OH)\text{-}CR^3(OH)CH(OH)\text{-}R^4$ (A-2) und a mol-% einer jodverbindung (Kat.) bezogen auf das eingesetzte Hydrazin versetzt. Die so erhaltene Mischung wurde anschließend t Stunden unter destillativer Entfernung von Wasser auf T°C erhitzt.

Nach üblicher Aufarbeitung erhielt man y % an Pyrazol, bezogen auf das eingesetzte Hydrazin bzw. dessen Derivat.

2.2
n g einer c Gew.-%igen Schwefelsäure wurden bei T°C gleichzeitig oder nacheinander mit einem Acrolein/Vinylalkylketon (A-1), bzw. einem (β-Hydroxy)ethylalkylketon $R^2\text{-}CH(OH)\text{-}CR^3(H)\text{-}CO\text{-}R^4$(A-1∗) bzw. ei-

nem Glycerin (A-2), Hydrazinhydrat oder einem Hydrazin (A-3), und a mol-% einer Jodverbindung (Kat.), bezogen auf das eingesetzte Hydrazin, versetzt. Nach t Stunden Gesamtreaktionszeit (Zugabe und Nachrühren) wurde wie üblich aufgearbeitet.

2.3

n g einer c Gew.-%igen Schwefelsäure wurden unter Kühlung gleichzeitig oder nacheinander mit Hydrazinhydrat oder einem Hydrazin (A-3) und a mol-% einer Jodverbindung (Kat.), bezogen auf das eingesetzte Hydrazin, versetzt. Die so erhaltene Mischung wurde auf T°C erhitzt und bei dieser Temperatur mit einem Acrolein/Vinylalkylketon bzw. einem (β-Hydroxy)ethylalkylketon $R^2$-CH(OH)-$CR^3$(OH)-$CR^3$(H)-CO-$R^4$(A-1∗) bzw. einem Glycerin (A-2) versetzt. Nach t Stunden Reaktionszeit (Zugabe von A-1, A-1 bzw. A-2 und Nachrühren) wurde wie üblich aufgearbeitet.

Die Einzelheiten der durchgeführten Versuche sind der nachstehenden Tabelle zu entnehmen.

Tabelle

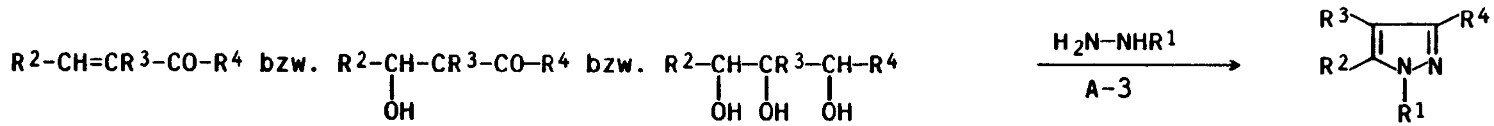


A-1 A-1* A-2

| Beispiel Nr. | A-1 bzw. A-2 R2 | R3 | R4 | mol | A-3 R1 | mol | $H_2SO_4/H_2O$ n[g]c) | c | Kat. Art | ac) | t[h] | T[°C] | y[%]c) | Pyrazol R1 | R2 | R3 | R4 | Verfahrens-variante |
|---|---|---|---|---|---|---|---|---|---|---|---|---|---|---|---|---|---|---|
| 2b) | H | H | H | 1,1 | H | 1,0 | 449,6 | 47,9 | NaJ | 1,34 | 4,5 | 145-155 | 86,6 | H | H | H | H | 2.1 |
| 3b) | H | H | H | 1,1 | H | 1,0 | 269,6 | 80,0 | NaJ | 0,67 | 2,5 | 155 | 91,8 | H | H | H | H | 2.2 |
| 4a) | H | H | H | 1,2 | H | 1,0 | 410 | 70,2 | NaJ | 0,67 | 3,0 | 120 | 76,7 | H | H | H | H | 2.3 |
| 5a) | H | H | H | 1,2 | H | 1,0 | 392,2 | 50,0 | NaJ | 0,67 | 2,5 | 155 | 82,0 | H | H | H | H | 2.1 |
| 6b) | H | H | H | 1,1 | H | 1,0 | 431,2 | 50,0 | $NaJO_3$ | 0,66 | 4,0 | 155 | 80,8 | H | H | H | H | 2.1 |
| 7b) | H | H | H | 1,1 | H | 1,0 | 431,2 | 50,0 | $CH_3J$ | 0,7 | 4,0 | 155 | 19,7 | H | H | H | H | 2.1 |
| 8b) | H | H | H | 1,1 | H | 1,0 | 269,6 | 80,0 | HJ | 0,66 | 2,5 | 155 | 82,3 | H | H | H | H | 2.2 |
| 6b) | H | H | H | 1,1 | H | 1,0 | 431,2 | 50,0 | $J_2$ | 0,17 | 2,0 | 155 | 78,5 | H | H | H | H | 2.2 |
| 10a) | H | H | H | 1,2 | $CH_3$ | 1,0 | 420,2 | 70,0 | NaJ | 1,34 | 2,0 | 118 | 57,1 | $CH_3$ | H | H | H | 2.3 |
| 11a) | H | $CH_3$ | H | 2,4 | H | 2,0 | 410,0 | 70,2 | NaJ | 0,67 | 3,0 | 115-130 | 85,4 | H | H | $CH_3$ | H | 2.3 |
| 12a) | $CH_3$ | H | H | 1,2 | H | 1,0 | 410,0 | 70,2 | NaJ | 0,67 | 3,0 | 115-130 | 75,6 | H | $CH_3$ | H | H | 2.3 |
| 13a) | H | H | $CH_3$ | 1,17 | H | 1,0 | 410,0 | 70,2 | NaJ | 0,67 | 2,5 | 115-130 | 91,2 | H | H | H | $CH_3$ | 2.3 |
| 14a) | H | $CH_3$ | H | 1,2 | $CH_3$ | 1,0 | 410,0 | 70,2 | NaJ | 0,67 | 3,0 | 130 | 59,4 | $CH_3$ | H | $CH_3$ | H | 2.3 |
| 15a)* | H | H | $CH_3$ | 1,1 | H | 1,0 | 431,2 | 50,0 | NaJ | 0,67 | 3,0 | 100-155° | 79,5 | H | H | H | $CH_3$ | 2.3 |

a) aus A-1; b) aus A-2; a)* aus A-1*; c) bezogen auf 1 mol A-3

Beispiel 15

Herstellung von 3-Methylpyrazol aus 3-Ketobutanol-1

431,2 g 50 %ige Schwefelsäure wurden innerhalb von 15 Minuten bei Raumtemperatur mit 50 g (1 mol) Hydrazinhydrat und 1 g Natriumiodid versetzt. Anschließend erhitzte man auf 100°C und ließ innerhalb von 60 Minuten 193,6 g (1,1 mol) 50 %ige wäßrige 3-Ketobutanol-1-Lösung zutropfen. Durch Abdestillieren von Wasser wurde bis zum Ende der Zugabe eine Reaktionstemperatur von 155°C erreicht, danach wurde bei dieser Temperatur 50 Minuten nachgerührt. Insgesamt wurden 350 g Wasser abdestilliert.

Nach Abkühlen auf 70°C wurde mit 15 %iger Natronlauge neutralisiert und mit 1,2-Dichlorethan extrahiert. Die vereinigten organischen Extrakte wurden über Natriumsulfat getrocknet, abfiltriert und am Rotationsverdampfer eingeengt. Man erhielt 90,4 g dunkelbraunes Öl, aus dem durch Destillation im Vakuum 65,2 g 3-Methylpyrazol vom $Kp_{35}$ 108°C erhalten wurden, welches durch GC-Vergleich mit authentischem Material indentifiziert wurde.

Beispiel 16

Zu 431,2 g (2,2 mol) 50 %iger Schwefelsäure tropfte man 50 g (1 mol) Hydrazinhydrat, wobei die Reaktionstemperatur auf 90°C anstieg. Nach Zugabe von 1 g Natriumjodid tropfte man innerhalb 30 Minuten bei 90°C eine Lösung von 90,4 g (1,1 mol) 36,5 %iger Formalinlösung und 63,8 g (1,1 mol) Aceton zu. Man erhöhte die Reaktionstemperatur innerhalb von 60 Minuten unter Abdestillieren von Wasser auf 153°C und rührte anschließend weitere 45 Minuten bei dieser Temperatur nach. Insgesamt wurden 330 g Wasser abdestilliert.

Nach Neutralisation mit 25 %iger Natronlauge wurde mit 1,2-Dichlorethan extrahiert. Die vereinigten 1,2-Dichlorethanextrakte wurden getrocknet und eingedampft. Man erhielt 28 g braunes Öl, dessen Gaschromatogramm 80 Flächenprozent 3-Methylpyrazol zeigte.

## Patentansprüche

1. Verfahren zur Herstellung von Pyrazolen der Formel

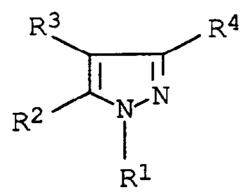

in der $R^1$ bis $R^4$ für Wasserstoff oder für $C_1$-$C_8$-Alkyl, $C_3$-$C_8$-Cycloalkyl, Aralkyl oder Aryl stehen, wobei diese Reste ihrerseits durch Heteroatome wie Stickstoff, Sauerstoff und Schwefel unterbrochen sein oder weitere Substituenten-wie Halogen, Nitro, Sulfonyl, $C_1$-$C_4$-Alkylsulfonyl, Arylsulfonyl und Carboxyl tragen können, durch Dehydrierung von 2-Pyrazolinen Ia

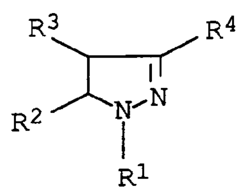

Ia

dadurch gekennzeichnet, daß man die Umsetzung mittels Schwefelsäure in Gegenwart von Jod oder einer Jod oder Jodwasserstoff freisetzenden Verbindung bei einer Temperatur von 50°C bis 250°C vornimmt.

2. Verfahren zur Herstellung von Pyrazolen nach Anspruch 1, dadurch gekennzeichnet, daß man das 2-Pyrazolin Ia durch Umsetzung von

a) Glycerinen

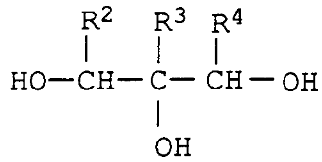

b) Acroleinen bzw. Vinylalkylketonen

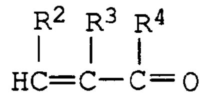

oder
c) β-Hydroxyethylalkylketonen

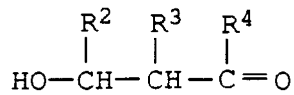

mit einem Hydrazin der Formel $R^1$-NH-$NH_2$ in situ im Reaktionsmedium erzeugt. $R^1$, $R^2$, $R^3$ und $R^4$ haben die in Anspruch 1 angegebene Bedeutung.

3. Verfahren nach den Ansprüchen 1 und 2, dadurch gekennzeichnet, daß man die Dehydrierung in Gegenwart von 0,01 bis 10 mol-% Jod bzw. einer Jod oder Jodwasserstoff freisetzenden Verbindung bezogen auf das Pyrazolin Ia bzw. das Hydrazin vornimmt.

4. Verfahren nach den Ansprüchen 1, 2 und 3, dadurch gekennzeichnet, daß man die Umsetzung mittels 30 bis 99 gew.-%iger Schwefelsäure vornimmt.

## Claims

1. A process for the preparation of a pyrazole of the formula

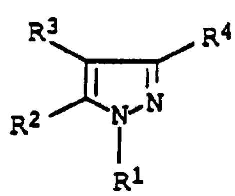

where $R^1$ to $R^4$ are each hydrogen or $C_1$-$C_8$-alkyl, $C_3$-$C_8$-cycloalkyl, aralkyl or aryl and these radicals may in turn be interrupted by hetero atoms, such as nitrogen, oxygen and sulfur, or may carry further substituents, such as halogen, nitro, sulfonyl, $C_1$-$C_4$-alkylsulfonyl, arylsulfonyl and carboxyl, by dehydrogenation of a 2-pyrazoline Ia

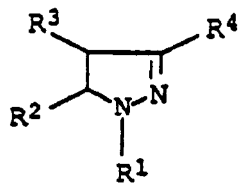

Ia

wherein the reaction is carried out using sulfuric acid in the presence of iodine or of an iodine or hydrogen iodide donor at from 50 to 250°C.

2. A process for the preparation of a pyrazole as claimed in claim 1, wherein the 2-pyrazoline Ia is produced by reacting
   a) a glycerol

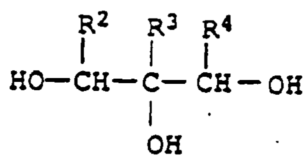

   b) an acrolein or vinyl alkyl ketone

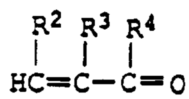

   or
   c) a β-hydroxyethyl alkyl ketone

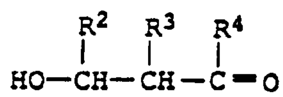

   with a hydrazine of the formula $R^1$-NH-$NH_2$ in situ in the reaction medium, $R^1$, $R^2$, $R^3$ and $R^4$ having the meanings stated in claim 1.

3. A process as claimed in claims 1 and 2, wherein the dehydrogenation is carried out in the presence of from 0.01 to 10 mol%, based on the pyrazoline Ia or the hydrazine, of iodine or of an iodine or hydrogen iodide donor.

4. A process as claimed in claims 1, 2 and 3, wherein the reaction is carried out using from 30 to 99% strength by weight sulfuric acid.

## Revendications

1. Procédé de préparation de pyrazoles de formule

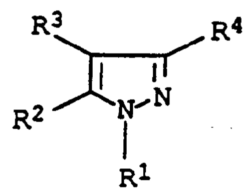

dans laquelle R1 à R4 sont mis pour hydrogène ou pour alkyle en C1-C8, cycloalkyle en C3-C8, aralkyle ou aryle, ces restes eux-mêmes pouvant être interrompus par des hétéroatomes tels que azote, oxygène et soufre ou pouvant porter d'autres substituants tels que halogène, nitro, sulfonyle, alkyle en C1-C4-sulfonyle, arylsulfonyle et carboxyle, par déshydrogénation de 2-pyrazolines Ia

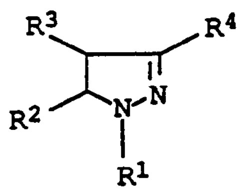

Ia

caractérisé par le fait que l'on effectue la réaction à une température de 50 ° C à 250 ° C, au moyen d'acide sulfurique, en présence d'iode ou de composé libérant de l'iode ou de l'acide iodhydrique.

2. Procédé de préparation de pyrazoles selon la revendication 1, caractérisé par le fait que l'on obtient in situ dans le milieu de réaction la 2-pyrazoline la par réaction de
a) glycérols

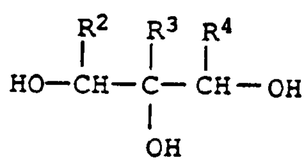

b) acroléines ou vinylalkylcétones

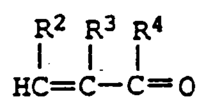

ou
c) p-hydroxyéthylalkylcétones

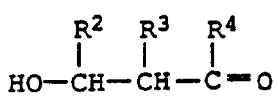

avec une hydrazine de formule R1-NH-$NH_2$.
R1, R2, R3 et R4 ayant la signification données dans la revendication 1.

3. Procédé selon la revendication 1 et 2, caractérisé par le fait que l'on effectue la déshydrogénation en présence de 0,01 à 10 mol % d'iode ou d'un composé libérant de l'iode ou de l'acide iodhydrique, proportion rapportée à la pyrazonline la ou l'hydrazine.

4. Procédé selon la revendication 1, 2 et 3, caractérisé par le fait que l'on effectue la réaction au moyen d'acide sulfurique à 30 à 99 % en poids.